# EUROPEAN PATENT APPLICATION

(11) **EP 4 122 436 A1**
(43) Date of publication of application: **25.01.2023**
(21) Application number: 21861077.2
(22) Date of filing: 26.07.2021
(51) Int. Cl.: A61F 13/15, A61F 13/51, A61F 13/511

(54) **ABSORBENT ARTICLE**

(30) Priority: 25.08.2020 JP 2020141654
(71) Applicant: Kao Corporation, Chuo-ku Tokyo 103-8210 (JP)
(72) Inventor: TOMITA, Mina, Haga-gun, Tochigi 321-3497 (JP); KOUTA, Takuya, Haga-gun, Tochigi 321-3497 (JP)
(74) Representative: Vossius & Partner Patentanwälte Rechtsanwälte mbB
(86) International application number: PCT/JP2021/027563
(87) International publication number: WO 2022/044649

(57) **Abstract**

An absorbent article according to one embodiment of the present invention has a sheet structure disposed on a surface layer side. The sheet structure comprises: a mixed fiber layer including synthetic fibers and natural fibers; and a synthetic fiber layer that is disposed on one surface side of the mixed fiber layer, mainly includes synthetic fibers as a fiber component, and constitutes the surface layer of the absorbent article. The mixed fiber layer includes a natural fiber lump including the aggregated natural fibers. The synthetic fiber layer includes a synthetic fiber lump including the aggregated synthetic fibers.

## Description

### Field of the Invention

The present invention relates to an absorbent article such as a disposable diaper or a sanitary napkin.

### Background of the Invention

There are known absorbent articles using natural fibers such as cotton from the viewpoint of giving a sense of security to a user and improving texture. For example, Patent Literature 1 discloses an absorbent article in which a topsheet is made of a cotton nonwoven fabric. Further, Patent Literature 2 discloses a topsheet in which a layer made of synthetic fibers is disposed on the skin side, and a mixed layer of cotton and synthetic fibers is disposed on the non-skin side.

### Citation List

### Patent Literature

Patent Literature 1: Japanese Patent Application Laid-open No. 2009-148328
Patent Literature 2: Japanese Patent Application Laid-open No. 2016-220986

### Summary of the Invention

An absorbent article according to an embodiment of the present invention has a sheet structure disposed on a surface layer side. The sheet structure comprises: a mixed fiber layer including synthetic fibers and natural fibers; and a synthetic fiber layer that is disposed on one surface side of the mixed fiber layer, mainly includes synthetic fibers as a fiber component, and constitutes the surface layer of the absorbent article. The mixed fiber layer includes a natural fiber lump including the aggregated natural fibers. The synthetic fiber layer includes a synthetic fiber lump including the aggregated synthetic fibers.

### Brief Description of the Drawings

[Fig. 1] Fig. 1 is a view showing an example of a disposable diaper as an embodiment of an absorbent article of the present invention, and is a schematic plan view of the skin abutting surface side (topsheet side) showing a state where elastic members of respective portions are extended and spread in a planar shape.
[Fig. 2] Fig. 2 is a schematic cross-sectional view cut along the line II-II of Fig. 1, showing an example in which a sheet structure of the present invention forms an exterior body of the disposable diaper.
[Fig. 3] Fig. 3 is a schematic cross-sectional view in a cross-section similar to Fig. 2, showing another configuration example of the disposable diaper, and showing an example in which the sheet structure of the present invention forms a topsheet of the disposable diaper.
[Fig. 4] Fig. 4 is a schematic cross-sectional view showing an example of the sheet structure of the present invention.
[Fig. 5] Fig. 5 is a schematic view showing a configuration example of the sheet structure having an uneven structure, in which A is a cross-sectional view of a case where the sheet structure is used as an exterior body of the disposable diaper, B is a cross-sectional view of a case where the sheet structure is used as a topsheet of the disposable diaper, and C is a plan view of a disposable diaper using the sheet structure.
[Fig. 6] Fig. 6 is a schematic view showing another configuration example of the sheet structure having an uneven structure, in which A is a cross-sectional view of the sheet structure, and B is a plan view of a disposable diaper using the sheet structure.
[Fig. 7] Fig. 7 is a schematic view showing another configuration example of the sheet structure having an uneven structure, in which A is a cross-sectional view of the sheet structure, and B is a plan view of a disposable diaper using the sheet structure.

### Detailed Description of the Invention

As in the invention disclosed in Patent Literature 1, natural fibers have excellent hygroscopicity and water vapor releasability and can be expected to have an effect of enhancing a soft texture as a sheet, but on the other hand, have a problem that aggregated lumps are likely to be formed therein. With such aggregated lumps of natural fibers located on the surface of the sheet, there has been a possibility that a foreign body feeling due to the aggregated lumps occurs and the touch feeling of the surface is lowered.

Meanwhile, in the topsheet disclosed in Patent Literature 2, in order to suppress the decrease in the touch feeling due to the aggregated lumps of cotton, the layer made of synthetic fibers is disposed on the skin side. However, cotton does not have thermal adhesiveness, and when a strong deformation force is applied to the topsheet, the aggregated lumps of cotton move to the skin-side surface of the topsheet, which may lead to an uncomfortable feeling.

The present invention relates to providing an absorbent article capable of achieving a good touch feeling while using natural fibers.

Hereinafter, an absorbent article of the present invention will be described with reference to the drawings, exemplifying a disposable diaper as an embodiment.

### [Overall Configuration of Disposable Diaper]

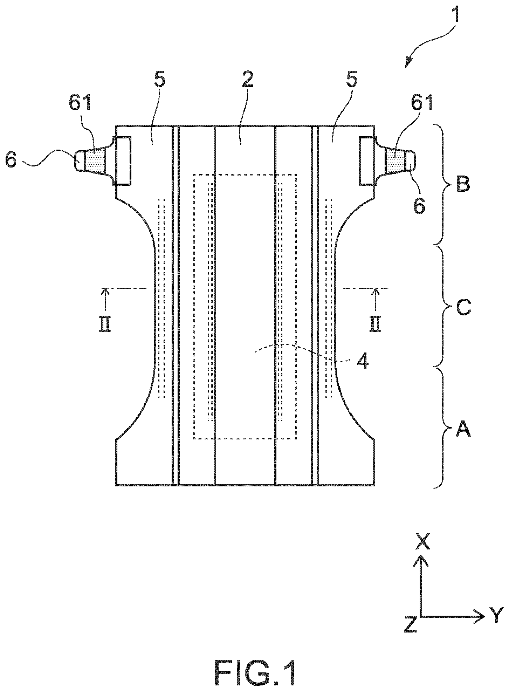

A disposable diaper 1 of this embodiment is a so-called unfolding-type disposable diaper as shown in Fig. 1. The disposable diaper 1 will be hereinafter referred to as a diaper 1.

The disposable diaper 1 has a longitudinal direction X corresponding to the front-back direction of a wearer and a lateral direction Y corresponding to the right-left direction of the wearer and orthogonal to the longitudinal direction X. In addition, the disposable diaper 1 has a thickness direction Z orthogonal to both the longitudinal direction X and the lateral direction Y. Those longitudinal direction X, lateral direction Y, and thickness direction Z are orthogonal to each other in a state where elastic members of respective portions are extended and spread in a planar shape.

In this specification, when each configuration is viewed from the thickness direction Z, it is referred to as "in plan view".

In this specification, regarding the thickness direction Z, when the disposable diaper is worn, the side closer to the skin of the wearer may be referred to as upper or skin side, and the side closer to the clothing may be referred to as lower or non-skin side. The phrase "when the diaper is worn" refers to the state where an adequate wearing position usually assumed is maintained.

The diaper 1 is divided into an abdominal region A located on the front (abdominal side) in the longitudinal direction X, a dorsal region B located on the rear (dorsal side) in the longitudinal direction X, and a crotch region C located between the abdominal region A and the dorsal region B.

The dorsal region B includes side portions that protrude outward in the lateral direction Y to the right and left from the crotch region C. The side edges of the side portions in the lateral direction Y are provided with fastening tapes 6.

Similarly, the abdominal region A includes side portions that protrude outward in the lateral direction Y to the right and left from the crotch region C. The non-skin-facing surface of the abdominal region A is provided with landing tapes (not shown) for bonding the fastening tapes 6. When the diaper is worn, the dorsal region B and the abdominal region A are bonded to each other by the fastening tapes 6 and integrally disposed around the low back and waist of the wearer.

The crotch region C includes leg openings that are formed to be constricted inward in the lateral direction Y such that the crotch region C has a narrower width than the abdominal region A and the dorsal region B. The crotch region C typically includes an excretion-part-facing portion that is disposed to face the excretion part (e.g., urination site) of the wearer when the diaper 1 is worn.

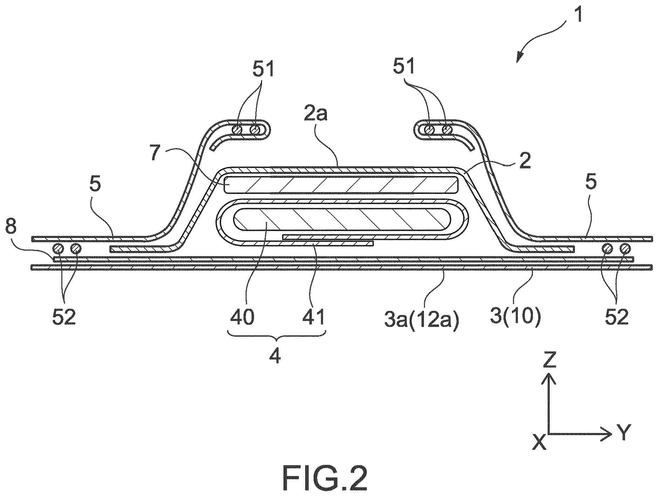

As shown in Figs. 1 and 2, the diaper 1 includes a topsheet 2, an exterior body 3, an absorbent member 4, side sheets 5, the pair of fastening tapes 6, an intermediate sheet 7, and a leakproof sheet 8. The diaper 1 has a configuration in which the exterior body 3, the leakproof sheet 8, the absorbent member 4, the intermediate sheet 7, and the topsheet 2 are laminated in the thickness direction Z. Those constituent elements are bonded to each other by, for example, publicly known bonding means such as a hot-melt adhesive.

The absorbent member 4 extends along the longitudinal direction X and is disposed between the topsheet 2 and the exterior body 3. The absorbent member 4 absorbs the wearer's liquid excreta such as urine (hereinafter, simply referred to as "liquid" sometimes) from its upper surface on the topsheet 2 side and diffuses the liquid inside, to retain the liquid.

The absorbent member 4 includes an absorbent core 40 and a core wrap sheet 41.

The absorbent core 40 includes, for example, a highly absorbent polymer capable of retaining the liquid. Specifically, the absorbent core 40 has a configuration formed of only a highly absorbent polymer, a configuration in which a fiber stack of hydrophilic fibers carries an absorbent polymer, or the like.

The core wrap sheet 41 covers the absorbent core 40 and has the function of retaining the shape of the absorbent core 40, for example. The core wrap sheet 41 is formed of, for example, thin and soft paper such as tissue paper or a liquid-permeable nonwoven fabric.

The topsheet 2 is disposed on the skin side of the absorbent member 4 (upper side in the thickness direction Z) and, for example, constitutes the central portion of the skin abutting surface of the diaper 1 in the lateral direction Y. The topsheet 2 is configured as a liquid-permeable sheet material and is formed of a woven fabric or nonwoven fabric made of synthetic fibers or natural fibers, a porous sheet, or the like. The topsheet 2 includes a skin abutting surface 2a that can abut on the skin of the wearer when the diaper is worn.

The intermediate sheet 7 is, for example, formed of a nonwoven fabric and disposed between the topsheet 2 and the core wrap sheet 41. The intermediate sheet 7 is disposed from the viewpoint of improving the permeability of the liquid from the topsheet 2 to the absorbent member 4, and the like.

The exterior body 3 is disposed on the non-skin side of the absorbent member 4 (lower side in the thickness direction Z), and for example, constitutes almost the entire non-skin-facing surface of the diaper 1 and constitutes the exterior of the diaper 1 when the diaper 1 is worn. The exterior body 3 includes an outer surface 3a that is the non-skin-facing surface of the diaper 1, and constitutes an outermost layer (layer closest to clothing side) of the diaper 1.

The pair of side sheets 5 are disposed at the side portions of the topsheet 2 in the lateral direction Y and constitute, for example, the side portions of the skin abutting surface of the diaper 1 in the lateral direction Y. The side sheets 5 are each formed of, for example, a sheet material having the functions of liquid-permeation-retardant property, water vapor permeability, water repellency, and the like. In each of the pair of side sheets 5, the central portion side thereof in the lateral direction Y is disposed to overlap with the topsheet 2, and the side portion thereof in the lateral direction Y extends to the outside of the topsheet 2 to be bonded to the exterior body 3.

The side sheet 5 may form three-dimensional gathers. A side end portion of the side sheet 5 on the central portion side in the lateral direction Y is a free end that is not bonded to the topsheet 2 or the like, and elastic members 51 are disposed therein. The elastic members 51 may extend in the longitudinal direction X in the crotch region C, and may extend to part of the abdominal region A and dorsal region B, for example. The elastic members 51 are provided to configure three-dimensional gathers.

In the vicinity of a side end portion of the side sheet 5, which is located outward in the lateral direction Y, for example, elastic members 52 stretchable in the longitudinal direction X are disposed to configure the leg gathers fitted around the legs of the wearer when the diaper is worn.

The elastic members 51 and 52 are thread-like or band-like elastic members stretchable in the longitudinal direction X.

The pair of fastening tapes 6 are provided at both side edges of the dorsal region B of the diaper 1 along the longitudinal direction X. The fastening tapes 6 include fastening portions 61 formed of male members of a mechanical hook-and-loop fastener. Further, the non-skin-facing surface in the abdominal region A of the diaper 1 includes fastened regions (not shown) formed of female members of the mechanical hook-and-loop fastener. The fastening portions 61 of the fastening tapes 6 can be detachably fastened to the fastened regions.

The leakproof sheet 8 is formed of a resin film having liquid impermeability or liquid-permeation-retardant property and is disposed on the skin side (upper side in the thickness direction Z) of the exterior body 3. The leakproof sheet 8 may have a function of moisture permeability for transmitting water vapor, for example.

### [Outline of Sheet Structure]

The diaper 1 of this embodiment includes the sheet structure 10 disposed on the surface layer side from the viewpoint of, for example, achieving a good touch feeling of a portion that comes into contact with the skin of a wearer or a person who replaces the diaper 1.

The "surface layer" of the diaper 1 means a portion including a skin-abutting surface, which can abut on the wearer's skin when the diaper is worn, and an outer surface disposed on the wearer's clothing side when the diaper is worn. In other words, the surface layer of the diaper 1 means a portion including a surface that can come into contact with the skin of the wearer or the skin of a person who replaces the diaper 1 or the like.

The skin abutting surface includes, for example, the skin abutting surface 2a of the topsheet 2. Further, if a part of the exterior body 3 is folded back to the skin side, that folded-back portion of the exterior body 3 also includes a skin abutting surface. Further, a portion of the three-dimensional gathers of the side sheet 5, which can touch the wearer's skin, also includes a skin abutting surface.

The outer surface is typically constituted by the outer surface 3a of the exterior body 3.

The phrase "the sheet structure 10 is disposed on the surface layer side" means that at least a part of the sheet structure 10 is disposed so as to include the skin abutting surface or the outer surface.

Specifically, the sheet structure 10 may constitute at least a part of the exterior body 3 or at least a part of the topsheet 2.

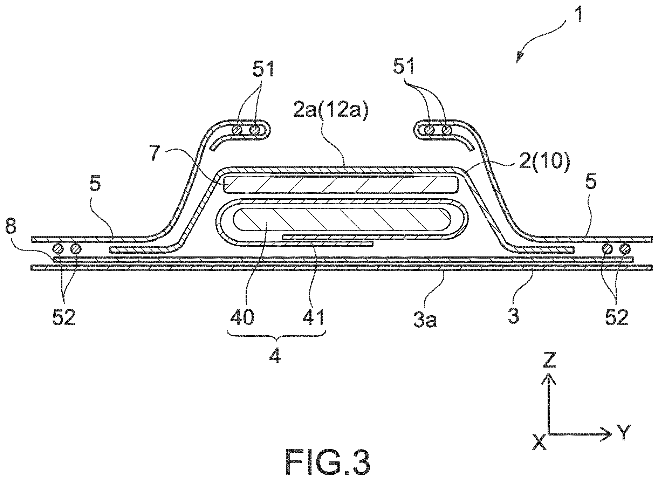

In the example shown in Fig. 2, the sheet structure 10 constitutes the exterior body 3. Alternatively, as shown in Fig. 3, the sheet structure 10 may constitute the topsheet 2.

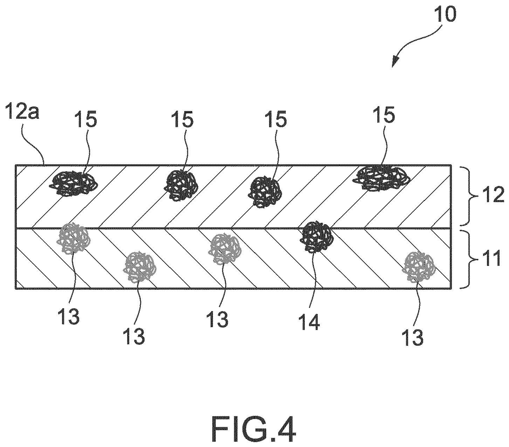

As shown in Fig. 4, the sheet structure 10 includes a mixed fiber layer 11 and a synthetic fiber layer 12 disposed on one surface side of the mixed fiber layer 11 and constituting the surface layer of the diaper 1. The phrase "the synthetic fiber layer 12 is disposed on one surface side of the mixed fiber layer 11" means the form in which the synthetic fiber layer 12 is preferably disposed on the mixed fiber layer 11, but the synthetic fiber layer 12 may be disposed on the mixed fiber layer 11 via another layer.

The synthetic fiber layer 12 includes a surface 12a. The surface 12a may constitute at least a part of the outer surface 3a of the exterior body 3 as shown in Fig. 2, or may constitute at least a part of the skin-abutting surface 2a of the topsheet 2 as shown in Fig. 3.

Note that, in Fig. 4, the cross-sections of the mixed fiber layer 11 and synthetic fiber layer 12 are shown by hatching of diagonal lines, but this hatching is for schematically showing the range of each layer and does not show the orientation of the fibers included in each of the layers or the like.

As will be described later, the mixed fiber layer 11 and the synthetic fiber layer 12 may be configured so as not to be separable and may form a single sheet material.

Alternatively, the mixed fiber layer 11 and the synthetic fiber layer 12 may form different sheet materials. In this case, the mixed fiber layer 11 and the synthetic fiber layer 12 may be bonded to each other by a hot-melt adhesive or the like. Further, the mixed fiber layer 11 and the synthetic fiber layer 12 are not limited to an example having the same shape in plan view. For example, the synthetic fiber layer 12 may be disposed only in a portion constituting the surface layer (skin abutting surface or outer surface) of the diaper 1 on the mixed fiber layer 11.

### [Configuration of Sheet Structure]

The mixed fiber layer 11 includes synthetic fibers and natural fibers and is configured as an aggregate of those fibers. The mixed fiber layer 11 is configured as a nonwoven fabric layer and includes, for example, one or more nonwoven fabric layers selected from an air-through nonwoven fabric, a spun lace nonwoven fabric, and a needle punch nonwoven fabric.

The natural fibers used in the mixed fiber layer 11 include one or more types of fibers selected from cotton, cellulose fibers obtained from natural materials other than cotton (hemp, palm, kraft pulp, straw, etc.), and the like. If the mixed fiber layer 11 includes natural fibers, it is possible to give a sense of security to consumers and also give a soft texture peculiar to natural fibers to the entire sheet structure 10.

In addition, natural fibers are more hygroscopic and more likely to release the water vapor retained in the nonwoven fabric than synthetic fibers. Hence, for example, if the sheet structure 10 is used as the exterior body 3, the natural fibers can absorb the water vapor derived from the moisture absorbed by the absorbent member 4 and further release the water vapor to the outside of the diaper 1. Further, for example, if the sheet structure 10 is used as the topsheet 2, the natural fibers can absorb the water vapor derived from excreted liquid or sweat of the wearer and release the water vapor to an unused portion of the absorbent member 4 (that is, a portion capable of absorbing the excreted liquid). Therefore, since the mixed fiber layer 11 includes natural fibers, it is possible to effectively suppress stuffiness when the diaper 1 is worn, and to suppress skin troubles of the wearer of the diaper 1.

The mixed fiber layer 11 preferably includes cotton as natural fibers. The cotton is particularly excellent in hygroscopicity and water vapor releasability as described above, and can more effectively suppress stuffiness when the diaper 1 is worn.

The mixed fiber layer 11 preferably includes natural fibers in an amount of 0.1 percent by mass or more, more preferably 0.5 percent by mass or more, and preferably 20 percent by mass or less, more preferably 10 percent by mass or less, from the viewpoint of obtaining the above-mentioned preferable action by the natural fibers and also controlling water retentivity by the natural fibers. If the natural fibers are included in an amount of the above-mentioned lower limit value or more, a soft texture peculiar to the natural fibers can be imparted to the entire sheet structure 10, and the effect of preventing stuffiness of the diaper 1 can be sufficiently obtained. If the natural fibers are included in an amount of the above-mentioned upper limit value or less, the water retentivity by the natural fibers can be regulated, and good liquid permeability can be obtained even when the sheet structure 10 is used as the topsheet 2, for example.

Further, the fineness of the natural fibers is, for example, preferably 1.0 tex or more, preferably 1.5 dtex or more, and preferably 15 dtex or less, preferably 10 dtex or less, from the viewpoint of preventing a natural fiber lump 13, which will be described later, from becoming harder due to the increase in the fiber density in the natural fiber lump 13 and also suppressing the deterioration of the texture due to the increase of a foreign body feeling when the natural fiber lump 13 becomes excessively large.

The fiber length of the natural fibers is, for example, preferably 5 mm or more, preferably 15 mm or more, and preferably 40 mm or less, preferably 35 mm or less, from the viewpoint of suppressing the fall-off of the natural fibers from the sheet structure 10 and the excessive increase in the size of the lump.

The mixed fiber layer 11 further includes a natural fiber lump 13 in which natural fibers are aggregated. The natural fiber lump 13 is a lamp formed when natural fibers are densely gathered. In the mixed fiber layer 11, for example, a plurality of natural fiber lumps 13 are disposed in a dispersed manner. Fig. 4 shows the natural fiber lump 13 as a configuration in which gray lines are densely gathered.

The shape and size of the natural fiber lump 13 in plan view are not particularly limited. For example, in the size of the natural fiber lump 13, a diameter (equivalent circle diameter) obtained when an area is calculated by image analysis or the like in plan view and converted into a perfect circle is preferably 0.3 mm or more, more preferably 0.5 mm or more, and preferably 5 mm or less, more preferably 2.5 mm or less.

The thickness of the natural fiber lump 13 is preferably 0.1 mm or more, more preferably 0.2 mm or more, and preferably 1.0 mm or less, more preferably 0.6 mm or less. Note that "the thickness of the natural fiber lump 13" means the dimension of the natural fiber lump 13 in the thickness direction of the sheet structure 10 (for example, in the thickness direction Z in Figs. 1 to 3).

Note that the size and thickness of the natural fiber lump 13 are calculated as the average value of the diameters and thicknesses of a total of 15 or more natural fiber lumps 13, for example, when the diameters and thicknesses of 5 or more natural fiber lumps 13 are measured in the mixed fiber layer 11 used for one diaper 1 (absorbent article), and the measurement is similarly performed on three diapers 1 (absorbent articles). The size and thickness of the natural fiber lump 13 may also be measured by coloring the natural fiber lump 13, for example, as will be described later in the section "Method of counting the number of fiber lumps".

The synthetic fibers included in the mixed fiber layer 11 include fibers made of, for example, polyolefin resins such as polyethylene and polypropylene, polyester resins such as polyethylene terephthalate, acrylic resins such as polyacrylic acid and polymethacrylic acid, vinyl resins such as polyvinyl chloride, polyamide resins such as nylon, and the like as raw materials. Among those various raw materials, a composite fiber (a sheath-core type composite fiber or a side-by-side type composite fiber) including a combination of two types of resins can also be used. Those synthetic fibers can be used singly or in combination of two types or more.

Of those, as the synthetic fibers included in the mixed fiber layer 11, a composite fiber is preferably used from the viewpoint of achieving both the strength of the nonwoven fabric and a soft and good tactile feeling.

The mixed fiber layer 11 preferably includes synthetic fibers in an amount of 80 percent by mass or more, more preferably 90 percent by mass or more, and preferably 99.8 percent by mass or less, more preferably 99.5 percent by mass or less, from the viewpoint of ensuring the strength of the mixed fiber layer 11 by combining the synthetic fibers and adjusting the water retentivity of the natural fibers or the like.

The fineness of the synthetic fibers of the mixed fiber layer 11 is, for example, preferably 0.5 dtex or more, preferably 1.0 dtex or more, and preferably 6.0 dtex or less, preferably 4.5 dtex or less, from the viewpoint of having a soft, fluffy tactile feeling.

The fiber length of the synthetic fibers of the mixed fiber layer 11 is, for example, preferably 20 mm or more, preferably 35 mm or more, and preferably 75 mm or less, preferably 70 mm or less, from the viewpoint of ease of forming webs when nonwoven fabrics are manufactured using a carding machine.

Note that, as shown in Fig. 4, the mixed fiber layer 11 may include a synthetic fiber lump 14 in which synthetic fibers are aggregated, in addition to the natural fiber lump 13. Fig. 4 shows the synthetic fiber lump 14 as a configuration in which black lines are densely gathered.

The synthetic fiber layer 12 mainly includes synthetic fibers as a fiber component and is configured as an aggregate of synthetic fibers. The synthetic fiber layer 12 includes, for example, synthetic fibers in an amount of 95 percent by mass or more, and may include synthetic fibers in an amount of 100 percent by mass. The synthetic fiber layer 12 is configured as a nonwoven fabric layer and includes, for example, one or more nonwoven fabric layers selected from an air-through nonwoven fabric, a spun-bonded nonwoven fabric, a spun lace nonwoven fabric, a melt-blown nonwoven fabric, a resin bonded nonwoven fabric, and a needle punched nonwoven fabric.

Note that the synthetic fiber layer 12 "mainly includes synthetic fibers as a fiber component" means that it is preferably formed only of synthetic fibers, but does not exclude that the synthetic fiber layer 12 includes natural fibers within the range in which a natural fiber lump is not formed.

The synthetic fibers included in the synthetic fiber layer 12 include fibers made of, for example, polyolefin resins such as polyethylene and polypropylene, polyester resins such as polyethylene terephthalate, acrylic resins such as polyacrylic acid and polymethacrylic acid, vinyl resins such as polyvinyl chloride, polyamide resins such as nylon, and the like as raw materials. Among those various raw materials, a composite fiber (a sheath-core type composite fiber or a side-by-side type composite fiber) including a combination of two types of resins can also be used. Those synthetic fibers can be used singly or in combination of two types or more.

Of those, as the synthetic fibers included in the synthetic fiber layer 12, a composite fiber having a fineness of 2.5 dtex or less is preferably used from the viewpoint of efficiency forming a synthetic fiber lump 15 to be described later.

The synthetic fibers included in the synthetic fiber layer 12 may be different from the synthetic fibers included in the mixed fiber layer 11, or may be at least partially the same. Further, the synthetic fiber layer 12 may include synthetic fibers having different fiber diameters and cross-sectional shapes.

The synthetic fiber layer 12 includes a synthetic fiber lump 15 in which synthetic fibers are aggregated. The synthetic fiber lump 15 is a lamp formed when synthetic fibers are densely gathered. In the synthetic fiber layer 12, for example, a plurality of synthetic fiber lumps 15 are disposed in a dispersed manner. Fig. 4 shows the synthetic fiber lump 15 as a configuration in which black lines are densely gathered.

The shape and size of the synthetic fiber lump 15 in plan view are not particularly limited. For example, in the size of the synthetic fiber lump 15, a diameter (equivalent circle diameter) obtained when an area is calculated by image analysis or the like in plan view and converted into a perfect circle is preferably 0.05 mm or more, more preferably 0.1 mm or more, and preferably 3 mm or less, more preferably 2 mm or less.

The thickness of the synthetic fiber lump 15 is preferably 0.05 mm or more, more preferably 0.08 mm or more, and preferably 0.5 mm less, more preferably 0.2 mm or less. Note that "the thickness of the synthetic fiber lump 15" means the dimension of the synthetic fiber lump 15 in the thickness direction of the sheet structure 10 (for example, in the thickness direction Z in Figs. 1 to 3).

Note that the size and thickness of the synthetic fiber lump 15 are calculated as the average value of the diameters and thicknesses of a total of 15 or more synthetic fiber lumps 15, for example, when the diameters and thicknesses of 5 or more synthetic fiber lumps 15 are measured in the synthetic fiber layer 12 used for one diaper 1 (absorbent article), and the measurement is similarly performed on three diapers 1 (absorbent articles).

The fineness of the synthetic fibers included in the synthetic fiber layer 12 is, for example, preferably 0.2 dtex or more, preferably 0.7 dtex or more, and preferably 2.5 dtex or less, preferably 2.0 dtex or less, from the viewpoint of efficiently forming the synthetic fiber lump 15.

The fiber length of the synthetic fibers is, for example, preferably 20 mm or more, preferably 35 mm or more, and preferably 75 mm or less, preferably 70 mm or less, from the viewpoint of efficiently forming the synthetic fiber lump 15.

Further, the fineness of the synthetic fibers included in the synthetic fiber layer 12 is preferably smaller than the fineness of the synthetic fibers included in the mixed fiber layer 11 from the viewpoint of a touch feeling.

The sheet structure 10 having the above configuration is produced as follows, for example.

For example, the synthetic fibers and natural fibers constituting the mixed fiber layer 11 are used to form a web. On the other hand, the synthetic fibers constituting the synthetic fiber layer 12 are used to form a web. In order to form webs, a publicly known device such as a carding machine can be used. The fibers included in those webs are combined by heat treatment or the like to form a mixed fiber layer 11 and a synthetic fiber layer 12. Subsequently, calendering or the like may be performed if necessary.

Examples of the treatment for combining fibers include an air-through method, a thermal bonding method, a needle punch method, and a spun lace method. From the viewpoint of efficiently forming the natural fiber lump 13 and the synthetic fiber lump 15, an air-through method is preferable.

Further, from the viewpoint of forming the synthetic fiber lump 15, fibers obtained by defibrating waste pieces of nonwoven fabrics may also be used for the web corresponding to the synthetic fiber layer 12, as the synthetic fibers serving as raw material. The waste pieces of nonwoven fabrics are generated, for example, in a manufacturing process of the nonwoven fabrics, when portions in both right and left side regions in the flow direction at the time of manufacturing are cut with a slitter. Such waste pieces are discarded usually, but in this embodiment, the regenerated fibers obtained by defibrating the waste pieces and returning them to fibers again can be used as synthetic fibers as raw material. For defibrating, for example, a publicly known defibrating machine can be used. Such regenerated fibers are usually in a state where combining (fusion bonding or the like) of the synthetic fibers remains. Therefore, use of the regenerated fibers makes it possible to efficiently form the synthetic fiber lump 15 in the synthetic fiber layer 12. For a specific manufacturing method, for example, Japanese Patent Application Laid-open No. 2013-151774 can be referred to.

Further, the number of fiber lumps formed into a web and the size thereof can be adjusted by adjusting the operating speed and the like of each constituent element of the carding machine. In particular, when the synthetic fiber layer 12 is formed using the regenerated fibers described above, the number of synthetic fiber lumps and the size thereof can also be adjusted by adjusting the ratio of the regenerated fibers in the entire synthetic fibers and the defibration degree.

Further, the treatment for combining the fibers may be performed in a state where the web forming the mixed fiber layer 11 and the web forming the synthetic fiber layer 12 are laminated. In this case, the synthetic fibers are interlaced with each other at the interface between the two layers, and a nonwoven fabric sheet having the laminate structure of the mixed fiber layer 11 and the synthetic fiber layer 12, which will be described later, is easily obtained.

Alternatively, the treatment for combining the fibers may be performed on each of the web forming the mixed fiber layer 11 and the web forming the synthetic fiber layer 12. In this case, a nonwoven fabric sheet corresponding to the mixed fiber layer 11 and a nonwoven fabric sheet corresponding to the synthetic fiber layer 12 are individually obtained.

Natural fibers are likely to be aggregated during web formation, and the natural fiber lump 13 is likely to be formed. Natural fibers including the natural fiber lump 13 are less likely to fuse with surrounding synthetic fibers. Hence, the natural fiber lump 13 easily moves when a force is applied to the sheet structure 10, for example.

On the other hand, the synthetic fibers are easily combined (fused) to each other by heat treatment or the like. Thus, combining points (fusion points) are easily formed between the synthetic fibers in the synthetic fiber lump 15 and between the synthetic fibers of the synthetic fiber lump 15 and the surrounding synthetic fibers. Therefore, the position of the synthetic fiber lump 15 is likely to be fixed in the synthetic fiber layer 12.

In the sheet structure 10 of this embodiment, the synthetic fiber layer 12 is disposed so as to constitute the surface layer of the diaper 1. As described above, even when a force is applied to the sheet structure 10, the position of the synthetic fiber lump 15 is likely to be fixed in the synthetic fiber layer 12, and thus the synthetic fiber lump 15 is prevented from protruding from the surface 12a. Therefore, it is possible to suppress a foreign body feeling of the surface layer of the diaper 1 and satisfactorily maintain a touch feeling.

Further, the synthetic fiber layer 12 in which the synthetic fibers are fused to each other is disposed on one surface side of the mixed fiber layer 11. Thus, the natural fiber lump 13 in the mixed fiber layer 11 is less likely to move to the surface layer side. In particular, since the synthetic fiber layer 12 includes the synthetic fiber lump 15 fused with the surrounding fibers, the movement of the natural fiber lump 13 toward the surface layer side is effectively limited by the synthetic fiber lump 15. This prevents the natural fiber lump 13 from protruding from the surface layer (the surface 12a of the synthetic fiber layer 12) of the diaper 1. Therefore, it is possible to suppress a foreign body feeling of the surface layer of the diaper 1 or the deterioration of a touch feeling due to the protrusion of the natural fibers, and it is possible to obtain the diaper 1 having a good touch feeling.

As described above, according to the sheet structure 10 of this embodiment, it is possible to achieve both a sense of security, a soft texture, and the effect of suppressing stuffiness by the natural fibers, and a good touch feeling of the surface layer of the diaper 1.

As shown in Fig. 2, if the sheet structure 10 is used as the exterior body 3, the surface 12a of the synthetic fiber layer 12 constitutes the outer surface 3a of the exterior body 3. Thus, for example, a person who operates the diaper 1, e.g., replaces or discards the diaper 1, can obtain a good touch feeling. Therefore, such a person can have a good impression to the diaper 1 together with a sense of security due to the natural fibers. In addition, when the exterior body 3 includes a portion folded back to the skin side, it is possible to improve a touch feeling of the folded-back portion and to suppress a stimulus to the skin due to the protrusion of the fibers during wearing of the diaper 1.

As shown in Fig. 3, if the sheet structure 10 is used as the topsheet 2, the surface 12a of the synthetic fiber layer 12 constitutes the skin abutting surface 2a of the topsheet 2. This makes it possible to suppress a foreign body feeling and a stimulus to the skin due to the protrusion of the fibers from the skin abutting surface 2a during wearing of the diaper 1. Therefore, the diaper 1 can give the wearer a good feeling of wearing and can also suppress skin troubles caused by the protrusion of the fibers.

From the viewpoint of satisfactorily suppressing the protrusion of the natural fibers from the surface 12a, the thickness of the synthetic fiber layer 12 is preferably 0.1 mm or more, more preferably 0.2 mm or more, and preferably 1.5 mm or less, more preferably 1.0 mm or less. Further, the thickness of the mixed fiber layer 11 is preferably equal to or less than the thickness of the natural fiber lump 13, particularly less than the thickness thereof.

From the viewpoint of obtaining a soft texture, good hygroscopicity, and water vapor releasability of the natural fibers, the thickness of the mixed fiber layer 11 is preferably 0.1 mm or more, more preferably 0.2 mm or more, and preferably 2.0 mm or less, more preferably 1.5 mm or less.

From the viewpoint of taking advantage of the texture of the natural fibers and also obtaining sufficient strength as the sheet structure 10 constituting the surface layer of the diaper 1, the thickness of the sheet structure 10 is preferably 0.3 mm or more, more preferably 0.5 mm or more, and preferably 3.0 mm or less, more preferably 2.0 mm or less.

From a similar viewpoint, the basis weight of the sheet structure 10 is preferably 10 g/m² or more, more preferably 15 g/m² or more, and preferably 60 g/m² or less, more preferably 30 g/m² or less.

### [Laminate Structure]

The mixed fiber layer 11 and the synthetic fiber layer 12 may have a laminate structure. The laminate structure used herein means that the synthetic fiber layer 12 is laminated on the mixed fiber layer 11 to constitute a nonwoven fabric sheet such that the two layers cannot be separated from each other. This makes it possible to handle the two layers as an integrated nonwoven fabric sheet, which facilitates handling of the diaper 1 during the manufacturing process.

Such a sheet structure 10 may be formed, for example, by performing treatment of combining fibers in a state where the web forming the mixed fiber layer 11 and the web forming the synthetic fiber layer 12 are laminated. Alternatively, the sheet structure 10 may be formed by forming a nonwoven fabric from the web forming the mixed fiber layer 11, forming a nonwoven fabric from the web forming the synthetic fiber layer 12, and then bonding those nonwoven fabrics to each other by an adhesive such as a hot-melt adhesive. Alternatively, a portion of the nonwoven fabric corresponding to the mixed fiber layer 11 and a portion of the nonwoven fabric corresponding to the synthetic fiber layer 12 may be bonded to each other by squeezing or the like.

In the case of a method of combining fibers of the webs corresponding to the respective layers, the synthetic fibers of the respective layers may be interlaced (combined) at the interface between the mixed fiber layer 11 and the synthetic fiber layer 12. Accordingly, even when the natural fiber lump 13 is disposed in the vicinity of the interface, it is possible to prevent the peeling between the mixed fiber layer 11 and the synthetic fiber layer 12 from occurring starting from the natural fiber lump 13.

In particular, as shown in Fig. 4, when the synthetic fiber lump 14 of the mixed fiber layer 11 is disposed at the interface between the mixed fiber layer 11 and the synthetic fiber layer 12, the synthetic fiber lump 14 may be combined with the synthetic fibers of both the mixed fiber layer 11 and the synthetic fiber layer 12. Therefore, it is possible to more reliably suppress the occurrence of peeling between the mixed fiber layer 11 and the synthetic fiber layer 12.

### [Flat Fibers]

The synthetic fiber layer 12 preferably includes synthetic fibers having a flattening ratio of 1.2 or more from the viewpoint of further improving a touch feeling of the surface layer of the diaper 1. In this specification, synthetic fibers having a flattening ratio of 1.2 or more are also referred to as "flat fibers".

The flattening ratio means the ratio of the major axis length to the minor axis length of the cross-section of the fiber (major axis length/minor axis length). When the cross-section of the fiber is a true circular shape, the flattening ratio is 1. As the degree of crush of the cross-section increases, the flattening ratio becomes larger than 1. A detailed method of measuring the flattening ratio of the fibers will be described later.

In the synthetic fiber layer 12, the major axis direction of the flat fibers may be oriented in a planar direction (a direction orthogonal to the thickness direction Z) or a direction nearly parallel to the planar direction. Such flat fibers are disposed in the vicinity of the surface 12a of the synthetic fiber layer 12, and thus the flat surfaces of the flat fibers can be disposed side by side on the surface layer of the diaper 1. As a result, the surface layer of the diaper 1 becomes more flat and more continuous than the case where the fibers having a cross-section with the shape close to a perfect circle are disposed on the surface 12a of the synthetic fiber layer 12 (the surface layer of the diaper 1), and a touch feeling of the surface layer of the diaper 1 can be further improved.

From the viewpoint described above, it is preferable that the flat fibers are disposed at least on the surface 12a of the synthetic fiber layer 12.

Further, since the fibers can be densely disposed in the planar direction in the synthetic fiber layer 12 by the flat fibers, the migration of the natural fiber lump 13 to the surface 12a is more restricted. This makes it possible to more effectively suppress the protrusion of the natural fiber lump 13 from the surface 12a, and more reliably enhance a touch feeling of the surface layer of the diaper 1.

Examples of the method of obtaining fibers having a flattening ratio of 1.2 or more include 1) a method of spinning using a spinneret having a flattening ratio corresponding to a desired flattening ratio or 2) a method of subjecting the deposition of fibers obtained using a normal spinneret (having a cross-section with the shape of a perfect circle or a cross-section with the shape similar thereto) to post-processing such as pressurizing. From the viewpoint of efficiently orienting the major axis direction of the flat fibers in the planar direction of the synthetic fiber layer 12, the method 2) is particularly preferable in this embodiment. The method 2) described above can be performed by, for example, pressurizing and compacting the synthetic fiber layer 12 including fibers having a circular cross-section while heating the synthetic fiber layer 12 as necessary. Examples of such treatment include calendering. The calendering is treatment in which heat and pressure are applied to a fiber aggregate such as a nonwoven fabric by a calendering roll to densify the fiber aggregate. By such calendering, flat fibers are formed in the surface 12a of the synthetic fiber layer 12, and the overlap of the synthetic fibers in the vicinity of the surface 12a of the synthetic fiber layer 12 is loosened, so that the flexibility of the synthetic fiber layer 12 can be enhanced.

### [Relationship of Fibers between Synthetic Fiber Layer and Mixed Fiber Layer]

From the viewpoint of further improving the touch feeling of the surface layer of the diaper 1 and preventing the natural fibers from falling off from the surface 12a of the synthetic fiber layer 12, it is preferable that the fiber diameters of the synthetic fibers of the synthetic fiber layer 12 and the mixed fiber layer 11 have the following relationship.

The fiber diameter of the synthetic fiber of the synthetic fiber layer 12 is preferably smaller than the fiber diameter of the synthetic fiber of the mixed fiber layer 11.

For example, the ratio of the fiber diameter of the synthetic fiber of the synthetic fiber layer 12 to the fiber diameter of the synthetic fiber of the mixed fiber layer 11 is preferably 0.95 or less, more preferably 0.8 or less, and preferably 0.05 or more, more preferably 0.1 or more.

The fiber diameter of the synthetic fiber of the synthetic fiber layer 12 is preferably 4 um or more, more preferably 8 um or more, and preferably 17 um or less, more preferably 15 um or less.

The fiber diameter of the synthetic fiber of the mixed fiber layer 11 is preferably 7 um or more, more preferably 10 um or more, and preferably 26 um or less, more preferably 22 um or less.

Note that, if the synthetic fiber layer 12 includes the flat fibers, the fiber diameter of the synthetic fiber can be measured as follows. If the flat fibers are distributed in a portion of the synthetic fiber layer 12, the fiber diameter of the synthetic fibers in a portion not including the flat fibers is measured. If the flat fibers are distributed throughout the synthetic fiber layer 12, the cross-sectional area of the cross-section of the fiber is measured by a method to be described later, and a diameter obtained in terms of a perfect circle is calculated.

Further, the fiber diameter of the synthetic fibers constituting the synthetic fiber lump 15 of the synthetic fiber layer 12 located on the surface layer side of the diaper 1 is preferably smaller than the fiber diameter of the natural fibers constituting the natural fiber lump 13 from the viewpoint of further improving a touch feeling of the surface layer of the diaper 1.

For example, the ratio of the fiber diameter of the synthetic fibers constituting the synthetic fiber lump 15 to the fiber diameter of the natural fibers constituting the natural fiber lump 13 is preferably 0.95 or less, and more preferably 0.8 or less.

The fiber diameter of the natural fibers of the mixed fiber layer 11 is preferably 9 um or more, more preferably 12 um or more, and preferably 36 um or less, more preferably 30 um or less.

Further, from the viewpoint of preventing the natural fiber lump 13 from protruding or falling off from the surface 12a of the synthetic fiber layer 12, it is preferable that the number of synthetic fiber lumps 15 per predetermined area in the synthetic fiber layer 12 located on the surface layer side of the diaper 1 is larger than the number of natural fiber lumps 13 per predetermined area in the mixed fiber layer 11.

The number of synthetic fiber lumps 15 per 100 cm² in the synthetic fiber layer 12 is preferably 10 or more, more preferably 20 or more, and preferably 400 or less, and more preferably 200 or less.

The number of natural fiber lumps 13 per 100 cm² in the mixed fiber layer 11 is preferably 5 or more, more preferably 10 or more, and preferably 200 or less, more preferably 150 or less.

From the viewpoint of more effectively reducing a foreign body feeling caused by the natural fiber lumps and improving a tactile feeling of the sheet surface, it is preferable that the size of the synthetic fiber lump 15 is smaller than the size of the natural fiber lump 13, in addition to the number of synthetic fiber lumps 15 per predetermined area in the synthetic fiber layer 12 being larger than the number of natural fiber lumps 13 per predetermined area in the mixed fiber layer 11.

The ratio of the size of the synthetic fiber lump 15 in the synthetic fiber layer 12 to the size of the natural fiber lump 13 in the mixed fiber layer 11 is preferably 0.8 or less, more preferably 0.65 or less. Note that the size of each fiber lump means an equivalent circle diameter in plan view.

From the viewpoint of more effectively reducing a foreign body feeling caused by the natural fiber lump 13 and achieving both a fluffy tactile feeling of the entire sheet and a smooth tactile feeling of the sheet surface, the thickness of the synthetic fiber lump 15 in the synthetic fiber layer 12 is preferably smaller than the thickness of the natural fiber lump 13 in the mixed fiber layer 11, and the thickness of the synthetic fiber layer 12 is preferably smaller than the thickness of the mixed fiber layer 11.

The ratio of the thickness of the synthetic fiber lump 15 in the synthetic fiber layer 12 to the thickness of the natural fiber lump 13 in the mixed fiber layer 11 is preferably 0.8 or less, and more preferably 0.5 or less.

The ratio of the thickness of the synthetic fiber layer 12 to the thickness of the mixed fiber layer 11 is preferably 0.95 or less, more preferably 0.8 or less.

### [Skin Care Agent]

If the seat construction 10 is used for a portion that abuts on the wearer's skin, the surface 12a of the synthetic fiber layer 12 is to be brought into contact with the wearer's skin for a long time. In such a case, from the viewpoint of suppressing the skin troubles of the wearer, the synthetic fiber layer 12 may contain a skin care agent on the surface of the synthetic fibers. A skin care agent is one that exhibits a skin care effect. The "skin care effect" means the overall efficacy to normalize a skin condition, such as skin rash prevention, anti-inflammation, wound prevention, and antimicrobial effect.

The skin care agent may be applied to the surface 12a of the synthetic fiber layer 12 after the formation of the synthetic fiber layer 12, or the synthetic fiber layer 12 may be formed using synthetic fibers to which the skin care agent is applied. The method of applying the skin care agent is not limited, and one or two or more methods selected from a die coater method, a slot spray method, a curtain spray method, a melt blown method, a spiral spray method, a gravure method, and a bead method can be appropriately selected. A detailed coating form of the skin care agent will be described later.

The skin care agent of this embodiment may contain, for example, a hydrophobic skin care agent from the viewpoint of being difficult to dissolve into liquid such as urine and obtaining a skin care effect for a long period. The hydrophobic skin care agent refers to one having no water solubility nor water dispersibility or having very poor dissolubility, and also refers to a composition or compound having efficacy of protection, healing, and the like for the skin of the wearer. For example, the hydrophobic skin care agent refers to a skin care agent that dissolves in an amount of less than 10 percent by mass after a sheet containing the skin care agent is immersed into water with an amount of 10 times as large as the mass of the sheet (liquid temperature: 25°C) and then left for 24 hours. The hydrophobic skin care agent is one preferably having solubility of 1 percent by mass or less, particularly preferably one in which the agent is not completely dissolved nor dispersed.

As the hydrophobic skin care agent, a natural extract component such as argan oil or shea butter, petrolatum, or the like can be used. For example, argan oil, which is a hydrophobic vegetable oil containing unsaturated fatty acids, maintains a balance between moisture and oil of the skin and prevents the skin from drying. In addition, the argan oil contains a large amount of unsaturated fatty acids such as oleic acid and linoleic acid, has a strong ability to remove active oxygen, and can reduce damage to the skin.

Further, the skin care agent of this embodiment may contain a hydrophilic skin care agent. The hydrophilic skin care agent refers to a skin care component having water solubility or water dispersibility, and is preferably one that suppresses the occurrence of rash and inflammation, and suppresses the progression of the rash and inflammation or alleviates the rash and inflammation when the rash and inflammation occur. The hydrophilic skin care agent refers to one having water solubility or water dispersibility, for example, which is dissolved or dispersed in an amount of 10 percent by mass or more after a sheet coated with a skin care agent is immersed into water with an amount of 10 times as large as the mass of the sheet (liquid temperature: 25°C) and then left for 24 hours. Note that the "dissolved or dispersed" amount described above is preferably 25 percent by mass or more, more preferably 50 percent by mass or more.

As the hydrophilic skin care agent, it is possible to use a natural extract component such as peach leaf extract or hamamelis extract, a polyhydric alcohol having 2 to 4 carbon chain atoms, and a hydrophilic compound having a function of skin care. For example, peach leaf extract (hydrophilic extract), which is a plant extract, has an antibacterial action and an antiinflammatory action.

The various skin care agents described above may be used alone or in combination of a plurality of types. For example, at least two types of skin care agent components having different degrees of hydrophilicity and hydrophobicity may be used.

Further, it is preferable to use a natural extract component for the skin care agent. This makes it possible to further suppress an allergic reaction or skin trouble of the wearer and also give a sense of security or a refreshing impression to consumers.

### [Sheet Structure Having Uneven Structure]

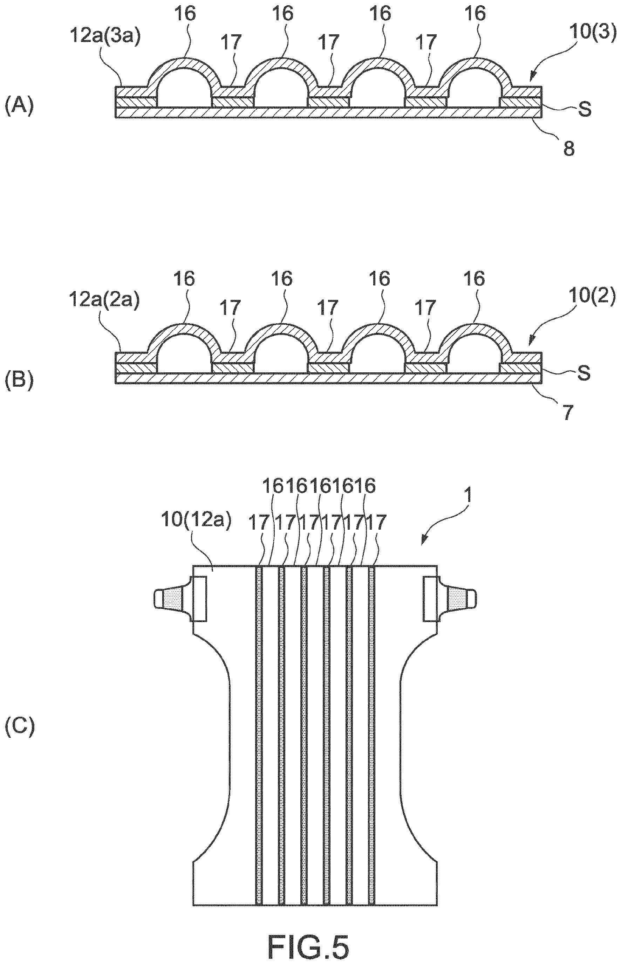

As shown in Figs. 5 to 7, the sheet structure 10 may include a plurality of protrusions 16 formed on the surface 12a of the synthetic fiber layer 12 and recesses 17 located between the plurality of protrusions 16.

For example, if the sheet structure 10 has the laminate structure, an uneven structure may be formed on the entire sheet structure 10.

If the synthetic fiber layer 12 and the mixed fiber layer 11 are formed of separate sheet materials, only the synthetic fiber layer 12 may have an uneven structure.

Since the sheet structure 10 has an uneven structure, particularly when the synthetic fiber layer 12 abuts on the skin, the contact area with the skin can be reduced, and the air permeability can be enhanced. Further, a touch feeling of the surface layer of the diaper 1 can be further improved.

The protrusion 16 means a portion protruding outward in the thickness direction Z of the diaper 1. The recess 17 means a portion formed around the protrusion 16. A detailed method of distinguishing the protrusion 16 and the recess 17 will be described later.

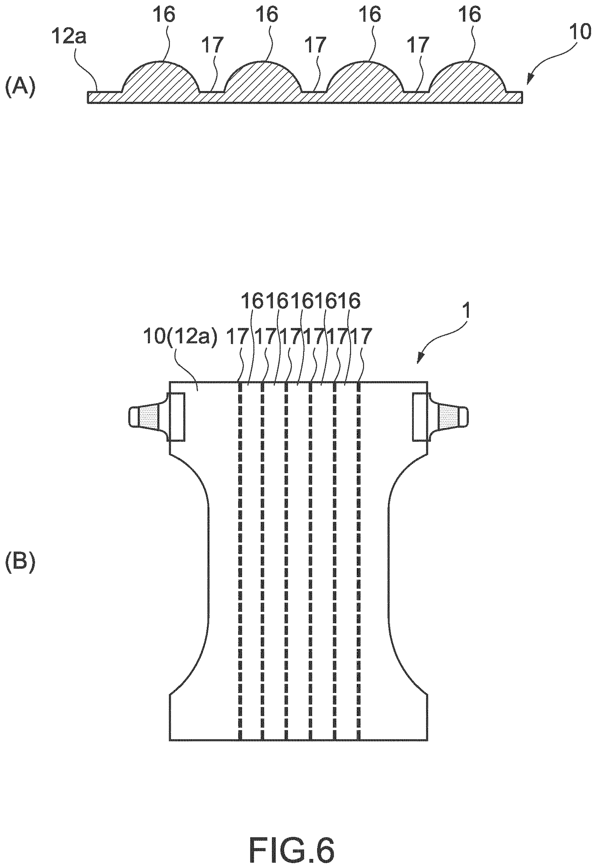

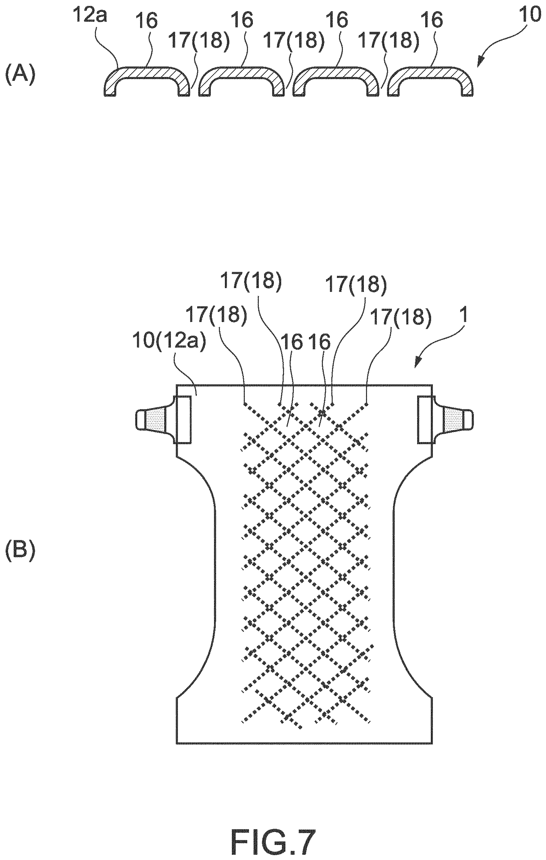

Configuration examples of the sheet structure 10 having an uneven structure will be described with reference to Figs. 5 to 7. However, the sheet structure 10 is not limited to those configurations. Note that, in the plan views of C of Fig. 5, B of Fig. 6, and B of Fig. 7, a portion indicated by a dot-pattern or lines represents the recess 17, and a portion adjacent to the recess 17 represents the protrusion 16.

In the sheet structure 10 shown in Fig. 5, an uneven structure is formed on the entire sheet structure 10, and the inside of the protrusion 16 is substantially hollow. The recess 17 is bonded to a sheet adjacent to the sheet structure 10 in the thickness direction Z.

In the example shown in A of Fig. 5, the sheet structure 10 constitutes the exterior body 3, and the recess 17 of the sheet structure 10 is bonded to the leakproof sheet 8 by an adhesive S such as a hot-melt adhesive.

In the example shown in B of Fig. 5, the sheet structure 10 constitutes the topsheet 2, and the recess 17 of the sheet structure 10 is bonded to the intermediate sheet 7 by an adhesive S such as a hot-melt adhesive.

Note that in addition to the configurations shown in A and B of Fig. 5, for example, the recess 17 of the synthetic fiber layer 12 having an uneven structure may be bonded to the substantially flat mixed fiber layer 11 to form the sheet structure 10 having an uneven structure.

The sheet structure 10 having such a configuration can be formed as follows. For example, after the web constituting the synthetic fiber layer 12 and the web constituting the mixed fiber layer 11 are laminated, the laminated webs may be disposed on a support having projections corresponding to the protrusions 16 and blown by hot air to provide the shape of the protrusions 16 and the recesses 17 (see, for example, Japanese Patent Application Laid-open No. 2013-177715). Alternatively, a nonwoven fabric constituting the sheet structure 10 may be bitten by the gear rolls to be stretched to provide the shape of the protrusions 16 and recesses 17 (see, for example, Japanese Patent Application Laid-open No. 2004-174234).

As shown in C of Fig. 5, the protrusions 16 and the recesses 17 may extend in one direction in plan view. The extending direction of the protrusions 16 and the recesses 17 may be the longitudinal direction X or the lateral direction Y, or may be a direction intersecting with the longitudinal direction X and the lateral direction Y. Further, the protrusions 16 and the recesses 17 are not limited to the form linearly extending, and may extend in a curved shape.

In the sheet structure 10 shown in A of Fig. 6, the inside of the protrusion 16 is substantially solid. Such a sheet structure 10 can be formed, for example, by embossing in the positions corresponding to the recesses 17 after the sheet structure 10 is formed. Examples of the embossing in the recesses 17 include publicly known embossing such as embossing with or without heat and ultrasonic wave embossing. As shown in B of Fig. 6, the embossed portions corresponding to the recesses 17 may extend intermittently or continuously in plan view. Further, as in the example shown in C of Fig. 5, the extending direction of the protrusions 16 and the recesses 17 is not limited to the example shown in B of Fig. 6.

In the sheet structure 10 shown in A of Fig. 7, the recess 17 includes a through-hole 18. This makes it possible to, in addition to providing a good touch feeling of the sheet structure 10, absorb or release water vapor, which is derived from excreted liquid, from the through-hole 18 and further improve air permeability.

Examples of the method of forming the through-hole 18 in the recess 17 include a method of melting the fibers and providing openings by adjusting the heat or pressure to be added when the recesses 17 are embossed, a method of melting a part of the fibers of the recesses 17 by applying ultrasonic vibration during embossing of the recesses 17, and a method of forming the through-holes 18 by perforating the recesses 17 with a convex pin. As specific methods, Japanese Patent Application Laid-open Nos. 2019-93598, 2011-110122, 2017-93731, and the like can be referred to.

In the pattern shown in B of Fig. 7, the recesses 17 and the through-holes 18 formed therein extend in two directions intersecting with the longitudinal direction X in plan view. In B of Fig. 7, the portions indicated by the lines represent the recesses 17 and the through-holes 18. In this case, the protrusions 16 are formed in a substantially rectangular region surrounded by the recesses 17 extending in two directions.

The planar patterns of the recesses 17 and the protrusions 16 are not limited to the examples shown in C of Fig. 5, B of Fig. 6, and B of Fig. 7. For example, in the case of forming the protrusions 16 as shown in Fig. 5, the protrusions 16 having various arrangements and shapes can be formed by changing the arrangements and shapes of the projections of the support during hot air blowing, the shape of the gear roll, and the like. Alternatively, in a case where the recesses 17 are formed by embossing or the like as shown in Fig. 6, the planar shape of the recess 17 is set to a dot shape (small circular shape), an elliptical shape, a rectangular shape, or the like, and the arrangement of such recesses 17 is changed, so that the protrusions 16 having various arrangements and shapes can be formed.

### [Supplementary Description]

Hereinafter, description of this embodiment will be additionally provided.

### (Method of Measuring Fiber Fineness)

A measurement sample cut out into a rectangular shape with the size of 50 mm by 100 mm (area is 5000 mm²) is prepared from the sheet structure 10 under no load. Next, in a cross-sectional view of the measurement sample, the fiber thickness of 10 fibers at a position spaced from one surface by 0.2 mm in the thickness direction is actually measured using an electron microscope, and a fiber thickness average value Dn (µm) is calculated. Next, the constituent components of the fibers at the position spaced from the one surface by 0.2 mm in the thickness direction are identified, and a theoretical fiber presence density Pn (g/cm³) is obtained using a differential scanning calorimeter (DSC). The weight (g) per 10,000 m of fiber length is calculated from the obtained fiber thickness average value Dn (µm) and theoretical fiber presence density Pn (g/cm³), and the calculated value is defined as the fineness (dtex) of the fibers.

Note that in the case where a measurement target such as the sheet structure 10 is taken out from the absorbent article, it is preferable to weaken the adhesive or the like used for bonding a constituent member to be measured and another constituent member by cooling means such as cold spray, and then carefully peel off the constituent member to be measured and take it out. This method can also be used for each measurement method and the like to be described below.

### (Method of Measuring Thickness of Sheet Structure)

The sheet structure 10 to be measured is cut into the size of 50 mm in the longitudinal direction by 50 mm in the lateral direction to prepare a cut piece of the sheet structure 10. Next, the cut piece is placed on a flat plate, a flat glass plate is placed thereon, a weight is evenly placed on the glass plate such that the load including the glass plate reaches 49 Pa, and then the thickness of the cut piece is measured. The measurement environment has a temperature of 20±2°C and a relative humidity of 6515%, and a microscope (manufactured by KEYENCE CORPORATION, VHX-1000) is used as a measuring device. To measure the thickness of the cut piece, first, an enlarged photograph of the cut surface of the cut piece is taken. This enlarged photograph simultaneously shows one having a known dimension. Next, the scale is adjusted to the enlarged photograph of the cut surface of the cut piece, and the thickness of the cut piece, that is, the thickness of the sheet to be measured is measured. The above operations are performed three times, and the average value of the operations performed three times is defined as the thickness of the sheet structure 10 to be measured or each layer. Note that if the sheet structure 10 to be measured has the laminate structure, the boundary thereof is determined from the difference in fiber diameter and/or fiber density, and the thickness of each layer is calculated.

### (Method of Measuring Basis Weight of Sheet Structure)

A portion of the sheet structure 10 to be measured is cut using a single-edged razor blade manufactured by Feather Safety Razor Co., Ltd., and a measurement piece is obtained so as to have a predetermined area. The weights of those measurement pieces are measured using an electronic analytical scale (electronic analytical scale GR-300 manufactured by A&D Company, Limited, accuracy: 4 decimal point places). The obtained weights are divided by the area of the measurement piece to calculate the basis weight of the measurement piece. The average of the basis weights of the five measurement pieces is defined as the basis weight.

### (Method of Measuring Fiber Diameter)

The measurement target (mixed fiber layer 11 and synthetic fiber layer 12) is cut with a razor blade (for example, a single-edged razor blade manufactured by Feather Safety Razor Co., Ltd.) to obtain a measurement piece having a rectangular shape (8 mm × 4 mm) in plan view. In cutting the measurement target, care is taken not to destroy the structure of the cut surface of the measurement piece formed by the cutting due to a pressure or the like at the time of cutting. Examples of a preferable method of cutting a measurement target include a method in which a measurement target is placed in liquid nitrogen and sufficiently frozen before cutting of the measurement target, and is then cut. Using a double-sided paper tape (manufactured by NICHIBAN Co., Ltd., NICETACK NW-15), the measurement piece is attached to a sample table. Next, the measurement piece is coated with platinum. For the coating, the ion sputtering device E-1030 model (trade name) manufactured by Hitachi Naka Electronics Co., Ltd. is used, and the sputtering time is 30 seconds. The cut surface of the measurement piece is observed at a magnification of 1000 times using a S-4000 field emission-type scanning electron microscope manufactured by Hitachi, Ltd. For example, if the measurement target has the laminate structure, the boundary of each layer is determined from the difference in fiber diameter and/or fiber density by the image captured by the electron microscope. For each fiber existing in each layer, the length of the fiber in the width direction with respect to the longitudinal direction thereof is measured in 10 fibers, and the average value thereof is defined as the fiber diameter.

### (Method of Measuring Flattening Ratio of Fibers)

In the manner similar to the "Method of Measuring Fiber Diameter" described above, a measurement piece is prepared, and the cut surface thereof is observed with an electron microscope. For example, the cut surface of the synthetic fiber layer 12 is observed, the thickness in the width direction (major axis length) and the thickness in the thickness direction (minor axis length) with respect to the longitudinal direction of the fibers are measured, and the major axis length is divided by the minor axis length to calculate the flattening ratio of one fiber. This measurement is performed for 10 fibers, and the average value is defined as the flattening ratio.

### (Method of Measuring Fiber Diameter in Case of Flat Fiber)

As described above, if the flat fibers are distributed throughout the synthetic fiber layer 12, the fiber diameter is measured as follows.

In the manner similar to the "Method of Measuring Fiber Diameter" described above, a measurement piece is prepared, and the cut surface thereof is observed with an electron microscope. The cross-sectional area of the cross-section of the observed fiber is measured using image processing software. The measured cross-sectional area is regarded as the cross-sectional area of a perfect circle, and the diameter in the case of the perfect circle is calculated. The diameter is calculated in the same manner for 10 fibers in the synthetic fiber layer 12, and the average value is defined as the fiber diameter.

### (Method of Counting Number of Fiber Lumps)

The sheet structure 10 is peeled off from the absorbent article by using cold spray, and a measurement piece of 10 centimeters square is cut out from the sheet structure 10. The cut measurement piece is disposed on a black mount, and the number of fiber lumps is visually counted. The counted number is defined as "a".

Subsequently, the measurement piece is immersed in colored water (e.g., an edible dye such as red No. 2), sandwiched between water-absorbing paper such as Kimtowel (manufactured by NIPPON PAPER CRECIA CO., LTD.) to absorb water, and then dried at 70°C for 24 hours. The number of colored fiber lumps is visually counted. The counted number is defined as "b".

The number of synthetic fiber lumps 15 per 100 cm² is (a-b).

The number of natural fiber lumps 13 per 100 cm² is b.

Measurement pieces are cut out from two locations for one absorbent article, and the measurement is performed for three absorbent articles. The average value of the measurement results from the total of six measurement pieces is calculated, and the number of synthetic fiber lumps 15 and the number of natural fiber lumps 13 per 100 cm² are obtained.

### (Method for Measuring Thickness of Fiber Lump)

A sharp blade is used to obtain a cut surface including the center portion of the synthetic fiber lump 15. The cut surface is observed using a microscope (manufactured by KEYENCE CORPORATION, VHX-1000) at a magnification of 100 to 200 times, and the thickness of the synthetic fiber lump 15 is measured. Similarly, the thickness of the colored natural fiber lump 13 is measured.

### (Form of Skin Care Agent)

The skin care agent may be applied as a surface treatment agent including a mixture containing a hydrophilic oil agent and a skin care agent. Alternatively, after a nonwoven fabric is formed using fibers coated with a hydrophilic oil agent, a surface treatment agent containing a skin care agent may be applied to the nonwoven fabric.

The hydrophilic oil agent is not particularly limited as long as it is a general hydrophilizing agent used in hygiene products. The hydrophilic oil agent generally has the action of stably attaching a surface treatment agent, which is liquid, to the fibers.

Further, the surface treatment agent may contain, in addition to the skin care agent, one having a function other than the skin care effect (for example, an antibacterial agent, a deodorant, or the like).

The surface treatment agent containing the skin care agent may include a polyhydric alcohol having 2 to 4 carbon chain atoms. Such a polyhydric alcohol particularly functions as a solvent for a hydrophilic component (hydrophilic oil agent, hydrophilic skin care agent, or the like) and can uniformly disperse those components. In addition, the polyhydric alcohol has not only a function as a solvent but also a moisturizing effect and an effect of improving lubricity, and can also function as a skin care agent. By improving the lubricity, friction between the skin and the nonwoven fabric can be reduced, and damage to the skin can be suppressed.

Examples of the polyhydric alcohol include 1,3-butylene glycol. The 1,3-butylene glycol is a moisturizing, liquid, water-soluble base component that is not sticky and maintains the moisturization of the skin with a smooth feel of use.

### (Method of Distinguishing Protrusion and Recess)

The back surface (surface opposite to the surface 12a) of the sheet piece (measurement piece) of the sheet structure 10 having an uneven structure is attached to a flat acrylic plate. The measuring piece to which the acrylic plate is attached is placed on a general ink pad (for example, UNISTAMP (trade name), red, manufactured by Mitsubishi Pencil Co., Ltd.) used for putting a rubber stamp or the like, with the front 12a side facing down. Next, the measurement piece is then pressurized with 1.2 kPa from above the acrylic plate, and the surface 12a of the measurement piece is inked. Next, the measurement piece is placed on a blank sheet with the inked surface 12a (inked surface) of the measurement piece facing down, and the measurement piece is pressurized with 1.2 kPa from above the acrylic plate to transfer the ink to the blank sheet. A portion (contact portion) of the blank sheet on which the ink is transferred is defined as a protrusion, and a portion (non-contact portion) on which the ink is not transferred is defined as a recess.

Hereinabove, the embodiment of the present invention has been described, but the present invention is not limited to the embodiment described above and can be variously modified without departing from the gist of the present invention.

For example, the sheet structure 10 may include another layer between the mixed fiber layer 11 and the synthetic fiber layer 12.

Alternatively, in the sheet structure 10, the synthetic fiber layer 12 may be disposed on both surfaces of the mixed fiber layer 11.

Further, the sheet structure 10 may be used for three-dimensional gathers of the side sheets 5, and the surface 12a of the synthetic fiber layer 12 may constitute a skin abutting surface.

For example, in the embodiment described above, the example of the unfolding-type disposable diaper has been described as an absorbent article, but the present invention is not limited thereto. The absorbent article of the present invention may be, for example, a pants-type disposable diaper, a urine-absorbing pad, a pantyliner, a sanitary napkin, or the like.
<1> An absorbent article, which has a sheet structure disposed on a surface layer side, the sheet structure comprising:
   a mixed fiber layer including synthetic fibers and natural fibers; and
   a synthetic fiber layer that is disposed on one surface side of the mixed fiber layer, mainly includes synthetic fibers as a fiber component, and constitutes the surface layer of the absorbent article, wherein
   the mixed fiber layer includes a natural fiber lump including the aggregated natural fibers, and
   the synthetic fiber layer includes a synthetic fiber lump including the aggregated synthetic fibers.
<2> The absorbent article according to <1>, wherein
   the mixed fiber layer and the synthetic fiber layer have a laminate structure.
<3> The absorbent article according to <1> or <2>, wherein
   the synthetic fiber layer includes synthetic fibers each having a flattening ratio of 1.2 or more.
<4> The absorbent article according to any one of <1> to <3>, wherein
   a fiber diameter of each of the synthetic fibers of the synthetic fiber layer is smaller than a fiber diameter of each of the synthetic fibers of the mixed fiber layer.
<5> The absorbent article according to <4>, wherein
   the fiber diameter of each of the synthetic fibers of the synthetic fiber layer is 4 um or more and 17 um or less, preferably 8 um or more and 15 um or less.
<6> The absorbent article according to <4> or <5>, wherein
   the fiber diameter of each of the synthetic fibers of the mixed fiber layer is 7 um or more and 26 um or less, preferably 10 um or more and 22 um or less.
<7> The absorbent article according to any one of <1> to <6>, wherein
   a fiber diameter of each of the synthetic fibers constituting the synthetic fiber lump is smaller than a fiber diameter of each of the natural fibers constituting the natural fiber lump.
<8> The absorbent article according to <7>, wherein
   a ratio of the fiber diameter of each of the synthetic fibers constituting the synthetic fiber lump to the fiber diameter of each of the natural fibers constituting the natural fiber lump is 0.05 or more and 0.95 or less, preferably 0.1 or more and 0.8 or less.
<9> The absorbent article according to any one of <1> to <8>, wherein
   the synthetic fiber layer includes a skin care agent on surfaces of the synthetic fibers.
<10> The absorbent article according to any one of <1> to <9>, wherein
   the sheet structure includes a plurality of protrusions formed on a surface of the synthetic fiber layer and recesses located between the plurality of protrusions.
<11> The absorbent article according to <10>, wherein
   each of the recesses includes a through-hole.
<12> The absorbent article according to any one of <1> to <11>, wherein
   the sheet structure constitutes at least a part of an exterior body, and
   the synthetic fiber layer constitutes at least a part of an outermost layer of the exterior body.
<13> The absorbent article according to any one of <1> to <11>, wherein
   the sheet structure constitutes at least a part of a topsheet including a skin abutting surface, and
   the synthetic fiber layer constitutes at least a part of the skin abutting surface.
<14> The absorbent article according to any one of <1> to <13>, wherein
   the mixed fiber layer includes the synthetic fibers in an amount of 80 percent by mass or more and 99.8 percent by mass or less, preferably 90 percent by mass or more and 99.5 percent by mass or less.
<15> The absorbent article according to any one of <1> to <14>, wherein
   a fineness of each of the synthetic fibers of the mixed fiber layer is 0.5 dtex or more and 6.0 dtex or less, preferably 1.0 dtex or more and 4.5 dtex or less.
<16> The absorbent article according to any one of <1> to <15>, wherein
   a fiber length of each of the synthetic fibers of the mixed fiber layer is 20 mm or more and 75 mm or less, preferably 35 mm or more and 70 mm or less.
<17> The absorbent article according to any one of <1> to <16>, wherein
   the synthetic fiber layer includes the synthetic fibers as a fiber component in an amount of 95 percent by mass or more, preferably 100 percent by mass.
<18> The absorbent article according to any one of <1> to <17>, wherein
   the synthetic fiber layer is free from the natural fiber lump.
<19> The absorbent article according to any one of <1> to <18>, wherein
   a fineness of each of the synthetic fibers included in the synthetic fiber layer is 0.2 dtex or more and 2.5 dtex or less, preferably 0.7 dtex or more and 2.0 dtex or less.
<20> The absorbent article according to any one of <1> to <19>, wherein
   the fineness of each of the synthetic fibers included in the synthetic fiber layer is smaller than the fineness of each of the synthetic fibers included in the mixed fiber layer.
<21> The absorbent article according to any one of <1> to <20>, wherein
   a fiber length of each of the synthetic fibers included in the synthetic fiber layer is 20 mm or more and 75 mm or less, preferably 35 mm or more and 70 mm or less.
<22> The absorbent article according to any one of <1> to <21>, wherein
   the number of the synthetic fiber lumps per predetermined area in the synthetic fiber layer is larger than the number of the natural fiber lumps per predetermined area in the mixed fiber layer.
<23> The absorbent article according to <22>, wherein
   a size of the synthetic fiber lump in the synthetic fiber layer is smaller than a size of the natural fiber lump in the mixed fiber layer.
<24> The absorbent article according to <23>, wherein
   a ratio of the size of the synthetic fiber lump in the synthetic fiber layer to the size of the natural fiber lump in the mixed fiber layer is preferably 0.8 or less, more preferably 0.65 or less.
<25> The absorbent article according to any one of <1> to <24>, wherein
   a thickness of the synthetic fiber lump in the synthetic fiber layer is larger than a thickness of the natural fiber lump in the mixed fiber layer.
<26> The absorbent article according to <25>, wherein
   a ratio of the thickness of the synthetic fiber lump in the synthetic fiber layer to the thickness of
   the natural fiber lump in the mixed fiber layer is 0.8 or less, preferably 0.5 or less.
<27> The absorbent article according to any one of <1> to <26>, wherein
   the thickness of the synthetic fiber layer is smaller than the thickness of the mixed fiber layer.
<28> The absorbent article according to <27>, wherein
   a ratio of the thickness of the synthetic fiber layer to the thickness of the mixed fiber layer is 0.95 or less, preferably 0.8 or less.

### Reference Signs List

- 1: disposable diaper (diaper, absorbent article)
- 2: topsheet
- 3: exterior body
- 10: sheet structure
- 11: mixed fiber layer
- 12: synthetic fiber layer
- 13: natural fiber lump
- 15: synthetic fiber lump

### Industrial Applicability

According to the absorbent article of the present invention, it is possible to achieve a good touch feeling while using natural fibers.

## Claims

1. An absorbent article, which has a sheet structure disposed on a surface layer side, the sheet structure comprising:
a mixed fiber layer including synthetic fibers and natural fibers; and
a synthetic fiber layer that is disposed on one surface side of the mixed fiber layer, mainly includes synthetic fibers as a fiber component, and constitutes the surface layer of the absorbent article, wherein
the mixed fiber layer includes a natural fiber lump including the aggregated natural fibers, and
the synthetic fiber layer includes a synthetic fiber lump including the aggregated synthetic fibers.

2. The absorbent article according to claim 1, wherein
the mixed fiber layer and the synthetic fiber layer have a laminate structure.

3. The absorbent article according to claim 1 or 2, wherein
the synthetic fiber layer includes synthetic fibers each having a flattening ratio of 1.2 or more.

4. The absorbent article according to any one of claims 1 to 3, wherein
a fiber diameter of each of the synthetic fibers of the synthetic fiber layer is smaller than a fiber diameter of each of the synthetic fibers of the mixed fiber layer.

5. The absorbent article according to claim 4, wherein
the fiber diameter of each of the synthetic fibers of the synthetic fiber layer is 4 um or more and 17 um or less.

6. The absorbent article according to claim 4 or 5, wherein
the fiber diameter of each of the synthetic fibers of the mixed fiber layer is 7 um or more and 26 um or less.

7. The absorbent article according to any one of claims 1 to 6, wherein
a fiber diameter of each of the synthetic fibers constituting the synthetic fiber lump is smaller than a fiber diameter of each of the natural fibers constituting the natural fiber lump.

8. The absorbent article according to claim 7, wherein
a ratio of the fiber diameter of each of the synthetic fibers constituting the synthetic fiber lump to the fiber diameter of each of the natural fibers constituting the natural fiber lump is 0.05 or more and 0.95 or less.

9. The absorbent article according to any one of claims 1 to 8, wherein
the synthetic fiber layer includes a skin care agent on surfaces of the synthetic fibers.

10. The absorbent article according to any one of claims 1 to 9, wherein
the sheet structure includes a plurality of protrusions formed on a surface of the synthetic fiber layer and recesses located between the plurality of protrusions.

11. The absorbent article according to claim 10, wherein
each of the recesses includes a through-hole.

12. The absorbent article according to any one of claims 1 to 11, wherein
the sheet structure constitutes at least a part of an exterior body, and
the synthetic fiber layer constitutes at least a part of an outermost layer of the exterior body.

13. The absorbent article according to any one of claims 1 to 11, wherein
the sheet structure constitutes at least a part of a topsheet including a skin abutting surface, and
the synthetic fiber layer constitutes at least a part of the skin abutting surface.

14. The absorbent article according to any one of claims 1 to 13, wherein
the mixed fiber layer includes the synthetic fibers in an amount of 80 percent by mass or more and 99.8 percent by mass or less.

15. The absorbent article according to any one of claims 1 to 14, wherein
a fineness of each of the synthetic fibers of the mixed fiber layer is 0.5 dtex or more and 6.0 dtex or less.

16. The absorbent article according to any one of claims 1 to 15, wherein
a fiber length of each of the synthetic fibers of the mixed fiber layer is 20 mm or more and 75 mm or less.

17. The absorbent article according to any one of claims 1 to 16, wherein
the synthetic fiber layer includes the synthetic fibers as a fiber component in an amount of 95 percent by mass or more.

18. The absorbent article according to any one of claims 1 to 17, wherein
the synthetic fiber layer is free from the natural fiber lump.

19. The absorbent article according to any one of claims 1 to 18, wherein
a fineness of each of the synthetic fibers included in the synthetic fiber layer is 0.2 dtex or more and 2.5 dtex or less.

20. The absorbent article according to any one of claims 1 to 19, wherein
the fineness of each of the synthetic fibers included in the synthetic fiber layer is smaller than the fineness of each of the synthetic fibers included in the mixed fiber layer.

21. The absorbent article according to any one of claims 1 to 20, wherein
a fiber length of each of the synthetic fibers included in the synthetic fiber layer is 20 mm or more and 75 mm or less.

22. The absorbent article according to any one of claims 1 to 21, wherein
the number of the synthetic fiber lumps per predetermined area in the synthetic fiber layer is larger than the number of the natural fiber lumps per predetermined area in the mixed fiber layer.

23. The absorbent article according to claim 22, wherein
a size of the synthetic fiber lump in the synthetic fiber layer is smaller than a size of the natural fiber lump in the mixed fiber layer.

24. The absorbent article according to claim 23, wherein
a ratio of the size of the synthetic fiber lump in the synthetic fiber layer to the size of the natural fiber lump in the mixed fiber layer is preferably 0.8 or less.

25. The absorbent article according to any one of claims 1 to 24, wherein
a thickness of the synthetic fiber lump in the synthetic fiber layer is larger than a thickness of the natural fiber lump in the mixed fiber layer.

26. The absorbent article according to claim 25, wherein
a ratio of the thickness of the synthetic fiber lump in the synthetic fiber layer to the thickness of the natural fiber lump in the mixed fiber layer is 0.8 or less.

27. The absorbent article according to any one of claims 1 to 26, wherein
the thickness of the synthetic fiber layer is smaller than the thickness of the mixed fiber layer.

28. The absorbent article according to claim 27, wherein
a ratio of the thickness of the synthetic fiber layer to the thickness of the mixed fiber layer is 0.95 or less.
